# EUROPEAN PATENT APPLICATION

(11) **EP 1 277 760 A1**
(43) Date of publication of application: **22.01.2003**
(21) Application number: 01925934.0
(22) Date of filing: 26.04.2001
(51) Int. Cl.: C07J 9/00, C07J 75/00, C07D 311/14, A61K 31/343, A61P 3/06, A61P 9/10

(54) **METHOD OF SEARCHING FOR ARTERIOSCLEROSIS INHIBITORS AND SHRINKERS**

(30) Priority: 28.04.2000 JP 2000129771; 18.05.2000 JP 2000146386; 13.07.2000 JP 2000212836
(71) Applicant: Takeda Chemical Industries, Ltd., Osaka-shi, Osaka 541-0045 (JP)
(72) Inventor: TOZAWA, Ryuichi, Toyonaka-shi, Osaka (JP); FUSE, Hiromitsu, Tsukuba-shi, Ibaraki 305-0821 (JP); KITA, Shunbun, Nishinomiya-shi, Hyogo 662-0823 (JP); NAKAMURA, Masahira, Kashiba-shi, Nara 639-0242 (JP)
(74) Representative: Lewin, John Harvey
(86) International application number: JP0103598
(87) International publication number: WO01083511

(57) **Abstract**

It is intended to find an efficient method of searching for drugs affecting the cholesteryl ester cycle and establish a system of efficiently searching for defoaming agents or regression agents artherosclerotic lesion and a system of searching for therapeutic agents remedies for atherosclerosis with a mechanism different from publicly known ones. A method of evaluating effects of a test substance on intracellular cholesteryl ester metabolic regulation which comprises selectively extracting free cholesterol with an aqueous alcohol from cells having been incubated in the presence of the test substance and cells having been incubated in the absence of the test substance, and then comparing the cholesteryl ester content in the cells having been incubated in the presence of the test substance with the cholesteryl ester content in the cells having been incubated in the absence of the test substance; in particular, a method of screening a compound having an activity of regulating the intracellular cholesteryl ester metabolism; and defoaming promoters, antiatherogenic agents or agents for regression of atherosclerotic lesion containing as the active ingredient compounds screened by the above method.

## Description

### Technical Field

The present invention relates to a method of searching for compounds useful as antiatherogenic agents and regression agents. More specifically, it relates to a searching method of efficiently evaluating and screening drugs for preventing or treating atherosclerosis as a major cause of ischemic heart diseases such as angina pectoris, myocardial infarction etc., and cerebral infraction ranked highest as a cause of death.

### Background Art

Studies on atherosclerosis have been conducted in light of pathogenic morphological features by using experimental atherosclerosis or human dissected preparations. A visual initial change in atherosclerosis lies in spot or linear fat deposition called fatty streak. Histologically, this change has been found to be attributable to accumulation of cells (foam cells) mainly composed of macrophages having cholesterol esters accumulated therein. Fatty streaks turn finally into fibrous plaques to be protruded from vascular walls, and infiltration thereof into smooth muscular cells, macrophages, T cells etc., cell necrosis images and accumulation of lipid are histologically recognized. As the morbid state further advances, the fibrous plaques accompanied by calcification and adhesion of thrombi cause vascular cavities to be narrow, or the plaques are ruined to cause thrombotic occlusion, and this is revealed to be closely related to occurrence of the acute coronary syndrome such as acute myocardial infarction, insecurity angina pectoris and ischemic sudden death (Fuster V et al., N. Engl. J. Med., 326, 242-250, 1992). It has also been revealed from a previous study that the readily ruined plaques have thin fibrous capsules, contain a large amount of lipid components such as cholesterol esters, and are instable (Erling F et al., Circulation, 92, 657-671, 1995), and therefore, removal of cholesterol esters, particularly cholesteryl esters from lesions, that is, defoaming, can be expected to stabilize instable plaques and to prevent them from being ruined, thus bringing about stabilization and regressione of atherosclerotic lesions thereby significantly reducing occurrence and recurrence of the acute coronary syndrome attributable to atherosclerosis.

As the intracellular cholesterol metabolic system involved in cellular foaming and defoaming, a metabolic cycle participating in exchange of free cholesterol with cholesteryl esters, that is, the cholesteryl ester cycle (Brown MS et al., J. Biol. Chem., 255, 9344-9352 (1980)) is known, and the turnover rate and equilibrated state of the cholesteryl ester cycle are reported to influence an activity of cellular foaming or defoaming (Hakamata H. et al., Arerioscr. Thromb., 14, 1860-1865 (1994)). The intracellular cholesteryl ester cycle is estimated to depend significantly on the activity of ACAT (Acyl-coenzyme A: cholesterol Acyltransferase) contributing to esterification of mainly free cholesterol and on the activity of neutral cholesteryl ester hydrolase contributing to hydrolysis of cholesteryl esters, but there are many unrevealed aspects in this regulatory mechanism. Accordingly, drugs regulating the cholesteryl ester cycle or drugs influencing formation and degradation of cholesteryl esters have the possibility of promoting cellular defoaming or regression of atherosclerotic lesions in a mechanism not known in the past, and are regarded as being effective as novel agents for treating atherosclerosis.

On one hand, quantification of intracellular cholesteryl esters has been conducted conventionally by a method of extracting the whole cell lipid with a mixture of organic solvents (for example, a chloroform-methanol mixture, a hexane-isopropanol mixture etc.) and then fractionating and quantifying the cholesteryl esters either by an enzyme method of using cholesterol oxidase or by TLC or liquid chromatography, but such methods are complicated to make highly sensitive measurement of many samples difficult, thus making it difficult to search for and evaluate drugs influencing the cholesteryl ester cycle.

### Summary of Invention

In light of the circumstances described above, an object of the present invention is to find a method of efficiently searching and evaluating drugs affecting the cholesteryl ester cycle and to establish:
(1) a screening system which can efficiently search the drugs for defoaming and regression of atherosclerotic lesion; and
(2) a screening system which can efficiently search for therapeutic agents for atherosclerosis with a mechanism different from known ones.

The present inventors made extensive study to solve the problem described above, and as a result they found that intracellular free cholesterol is selectively extracted and removed with an aqueous alcohol and/or cyclodextrins, and by utilizing this finding, a novel system of evaluating the intracellular cholesteryl ester metabolic regulation is established, thus enabling easy and highly sensitive measurement of an intracellular cholesteryl ester cycle activity, a cholesteryl ester-forming activity and a cholesteryl ester-degradation activity. By using this method, the present inventors further succeeded in constructing a system of searching for intracellular cholesteryl ester cycle regulators, cholesteryl ester formation inhibitors and cholesteryl ester degradation stimulators.

There is a report on an example where an alcohol was used in extraction of the whole lipid in cells and tissues, but there is no known example wherein an aqueous alcohol is used in selective extraction and removal of free cholesterol in order to directly quantify an activity on the intracellular cholesteryl ester metabolism. As examples wherein cyclodextrins are used in measurement of a reaction system or enzyme system participating in cholesterol metabolism, there are reports on measurement of the activities of ACAT and neutral cholesterol esterase in a cellular extract (Lisa M et al., Lipid, 31, 323-329 (1996)) and 7α-hydroxylase (Souidi M et al., Clin. Chim. Acta, 269, 201-217 (1998)), on measurement of transfer of cholesterol from cells (Kilsdonk EP et al., J. Biol. Chem., 270, 17250-17256 (1995)) and on fundamental data on extraction of intracellular free cholesterol (J. Pharmaceutical Science, 81, 521-523 (1992)), but there is no known example wherein cyclodextrin is used in direct evaluation of intracellular cholesteryl ester metabolic regulation.

The present invention has been completed on the basis of these findings, and provides:
(1) A method of extracting or removing intracellular free cholesterol which comprises selectively extracting or removing free cholesterol from cells with an aqueous alcohol;
(2) The extracting or removing method according to the above (1), wherein the alcohol is a polar aliphatic monohydric alcohol having 1 to 4 carbon atoms;
(3) The extracting or removing method according to the above (1), wherein the alcohol is methanol;
(4) The extracting or removing method according to the above (1), wherein the water content of the aqueous alcohol is 5 to 60 (v/v) %;
(5) The extracting or removing method according to the above (1), wherein the aqueous alcohol and cyclodextrin(s) are used;
(6) The extracting or removing method according to the above (1), wherein the cells are those derived from a mammal;
(7) The extracting or removing method according to the above (1), wherein the cells are peripheral blood-derived monocyte macrophages derived from a mammal, peritoneal macrophages derived from a mammal, alveolar macrophages derived from a mammal, aortic macrophages derived from a mammal, smooth muscular cells derived from a mammal, fibroblasts derived from a mammal, hepatocytes derived from a mammal, dendritic cell strains derived from a mammal, THP-1 (human), U937 (human), HepG2 (human), J774 (mouse), L-1 (mouse), RAW264.7 (mouse), WEHI (mouse) or P388D1 (mouse);
(8) A method of measuring the amount of intracellular cholesteryl esters which comprises selectively extracting and removing free cholesterol from cells with an aqueous alcohol and/or cyclodextrin(s) and then measuring the amount of the intracellular cholesteryl esters;
(9) The method according to the above (8), wherein whole lipid is extracted with a solvent from the cells from which free cholesterol has been extracted and removed, and the amount of cholesteryl esters in the whole lipid is quantified by an enzyme method using a cholesterol oxidase, a TLC method or liquid chromatography;
(10) The method according to the above (9), wherein the solvent is chloroform-methanol or hexane-isopropanol;
(11) The method according to the above (8), wherein the intracellular cholesterol is labeled, free cholesterol is selectively extracted and removed from the cells, and then the amount of the label in the cells is measured;
(12) A method of evaluating the regulatory activity of a test substance on the intracellular cholesteryl ester metabolism which comprises the procedure of selectively extracting free cholesterol from cells with an aqueous alcohol;
(13) The evaluation method according to the above (12), wherein (i) the cells are loaded with cholesterol or a cholesteryl ester, or (ii) a cholesteryl ester are formed in the cells to allow the cells to become foam cells;
(14) The evaluation method according to the above (13), wherein the loading of cells with cholesterol or a cholesteryl ester is carried out by addition of (i) a solution of cholesterol or a cholesteryl ester in an alcohol or (ii) a complex of cholesterol or a cholesteryl ester and lipoprotein;
(15) The evaluation method according to the above (14), wherein the lipoprotein is LDL, acetyl LDL, oxidized LDL, glycated LDL or β-VLDL;
(16) The evaluation method according to the above (13), wherein the formation of the cholesteryl ester in the cells is carried out by addition of oxysterols, lipoprotein, cholesterol-containing liposome or cytokine;
(17) The evaluation method according to the above (16), wherein the oxysterol is 25-hydroxycholesterol or 7-ketocholesterol;
(18) The evaluation method according to the above (16), wherein the lipoprotein is LDL, acetyl LDL, oxidized LDL, glycated LDL or β-VLDL;
(19) The evaluation method according to the above (16), wherein the cytokine is interferon γ;
(20) The evaluation method according to the above (12), wherein the alcohol is a polar aliphatic monohydric alcohol having 1 to 4 carbon atoms;
(21) The evaluation method according to the above (12), wherein the alcohol is methanol;
(22) The evaluation method according to the above (12), wherein the water content of the aqueous alcohol is 5 to 60 (v/v) %;
(23) The evaluation method according to the above (12), wherein the cells are those derived from a mammal;
(24) The evaluation method according to the above (12), wherein the cells are peripheral blood-derived monocyte macrophages derived from a mammal, peritoneal macrophages derived from a mammal, alveolar macrophages derived from a mammal, aortic macrophages derived from a mammal, smooth muscular cells derived from a mammal, fibroblasts derived from a mammal, hepatocytes derived from a mammal, dendritic cell strains derived from a mammal, THP-1 (human), U937 (human), HepG2 (human), J774 (mouse), L-1 (mouse), RAW264.7 (mouse), WEHI (mouse) or P388D1 (mouse);
(25) The evaluation method according to the above (12), wherein free cholesterol is selectively extracted with an aqueous alcohol from cells cultured in the presence of a test substance and cells cultured in the absence of the test substance, and then the amounts of intracellular cholesteryl esters in respective cells cultured in the presence of and in the absence of the test substance are measured and compared to each other;
(26) The evaluation method according to any one of the above (12) to (25), wherein the regulation of the intracellular cholesteryl ester metabolism is regulation of an activity of forming intracellular cholesteryl esters;
(27) The evaluation method according to any one of the above (12) to (25), wherein the regulation of the intracellular cholesteryl ester metabolism is regulation of an activity of hydrolysis of intracellular cholesteryl esters;
(28) The evaluation method according to any of the above (12) to (25) which is a method of screening a compound having an activity of regulating the intracellular cholesteryl ester metabolism;
(29) The evaluation method according to the above (28) which is a method of screening a compound having an inhibitory activity on formation of intracellular cholesteryl esters;
(30) The evaluation method according to the above (28) which is a method of screening a compound having a promoting activity on hydrolysis of intracellular cholesteryl esters;
(31) The evaluation method according to the above (28) which is a method of screening a compound having a defoaming activity;
(32) A compound having a regulatory activity on the intracellular cholesteryl ester metabolism selected by the method according to the above (28), or a salt thereof;
(33) A defoaming stimulator which comprises as an active ingredient a compound having a regulatory activity on the intracellular cholesteryl ester metabolism selected by the method according to the above (28), or a pharmaceutically acceptable salt thereof;
(34) Antiatherogenic agents or regression agents of atherosclerosis lesion which comprises as an active ingredient a compound having a regulatory activity on the intracellular cholesteryl ester metabolism selected by the method according to the above (28), or a pharmaceutically acceptable salt thereof;
(35) A method of stimulating defoaming which comprises administering an effective amount of a compound having a regulatory activity on the intracellular cholesteryl ester metabolism selected by the method according to the above (28), or a salt thereof to a mammal;
(36) A method of preventing and treating atherosclerosis which comprises administering an effective amount of a compound having a regulatory activity on the intracellular cholesteryl ester metabolism selected by the method according to the above (28), or a salt thereof to a mammal;
(37) Use of a compound having a regulatory activity on the intracellular cholesteryl ester metabolism selected by the method according to the above (28), or a salt thereof for the manufacture of a defoaming stimulator;
(38) Use of a compound having a regulatory activity on the intracellular cholesteryl ester metabolism selected by the method according to the above (28), or a salt thereof for the manufacture of an agent for preventing and treating atherosclerosis; and
(39) Use of an aqueous alcohol for selectively extracting or removing free cholesterol from cells.

### Detailed Description of the Invention

The aqueous alcohol used in the present invention may be any one containing water and an alcohol. The alcohol may be any polar alcohols, and polar aliphatic monohydric alcohols having 1 to 4 carbon atoms such as methanol, ethanol, isopropanol and butanol are preferable and may be used alone or in combination thereof. The water content of the aqueous alcohol can be, for example, 5 % to 60 % (v/v), preferably 10 % to 30 % (v/v). The aqueous alcohol used in the present invention may contain components other than water and alcohol insofar as selective extraction and removal of free cholesterol from cells are not adversely affected.

The cyclodextrin used in the present invention includes, for example, α-, β- or γ-cyclodextrins or derivatives thereof, and specifically, hydroxypropyl derivatives, methyl derivatives or saccharified derivatives such as hydroxypropyl α-cyclodextrin, hydroxypropyl β-cyclodextrin, hydroxypropyl γ-cyclodextrin, dihydroxypropyl β-cyclodextrin, methyl β-cyclodextrin and maltosyl β-cyclodextrin, are preferable and may be used alone or in combination thereof.

Cyclodextrin(s) are used usually in the form of an aqueous solution thereof, and the concentration of cyclodextrin(s) in the solution can be, for example, about 0.1 % (w/v) or more, preferably about 0.1 % to about 5 % (w/v), more preferably about 0.5 % to about 5 % (w/v).

The method of extracting or removing intracellular free cholesterol according to the present invention is characterized by selectively extracting and removing free cholesterol from cells with an aqueous alcohol.

The method of extracting or removing intracellular free cholesterol according to the present invention can also be carried out by using an aqueous alcohol and cyclodextrin(s). That is, the aqueous alcohol and cyclodextrin(s) may be simultaneously or successively used to remove free cholesterol from cells. The ratio of the aqueous alcohol to cyclodextrin(s) is not particularly limited within the range where the cyclodextrin(s) are dissolved, and when the both are simultaneously used, a cyclodextrin-containing aqueous alcohol may also be used.

More specifically, the method of extracting and removing intracellular free cholesterol according to the present invention is carried out by extracting objective cells with an aqueous alcohol or with an aqueous alcohol and cyclodextrin(s).

The objective cells are not particularly limited and may be any cells of a mammal including human beings, a plant, and a microorganism, and in particular cells derived from a mammal are preferable. The cells derived from a mammal include, for example, peripheral blood-derived monocyte macrophage, peritoneal macrophages, alveolar macrophages, aortic macrophages, smooth muscular cells, fibroblasts, hepatocytes and dendritic cell strains derived from mammals (human beings, monkeys, pigs, rabbits, rats, mice, hamsters etc.), and cell strains such as THP-1 (human), U937 (human), HepG2 (human), J774 (mouse), L-1 (mouse), RAW264.7 (mouse), WEHI (mouse), P388D1 (mouse) etc.

The extraction method is not particularly limited, and a method known per se, for example a method described by Hara A and Radin NS in Anal, Biochem. 90, 420-426 (1978) can be adopted, and the extraction conditions are also not particularly limited, and, usually, it is preferred that extraction is carried out at 15 to 45°C for about 3 minutes to 8 hours.

The method of measuring the amount of intracellular cholesteryl esters according to the present invention is carried out by selectively extracting and removing free cholesterol from cells with the above-described aqueous alcohol or cyclodextrin(s) and then measuring the amount of cholesteryl esters in the cells. Alternatively, the method of measuring the amount of intracellular cholesteryl esters according to the present invention can be carried out by using the aqueous alcohol and cyclodextrin(s).

The method of quantifying the amount of cholesteryl esters is not particularly limited, and for example, whole lipid is extracted with a suitable solvent such as chloroform-methanol or hexane-isopropanol from the cells from which free cholesterol has been removed, and the amount of cholesteryl esters in the whole lipid can be quantified by a method known per se, for example an enzyme method of using cholesterol oxidase, a TLC method, liquid chromatography, etc.

As described in the description hereinafter or in the Examples, quantification of cholesteryl esters is conducted preferably by selectively labeling intracellular cholesterol, extracting and removing free cholesterol from the cells, and measuring the amount of the label (e.g., radioactivity) in the cells.

The method of evaluating the activity of a test substance on regulation of the intracellular cholesteryl ester metabolism according to the present invention comprises the procedure of selectively extracting free cholesterol from cells with an aqueous alcohol. Alternatively, the method of evaluating the activity of a test substance on regulation of the intracellular cholesteryl ester metabolism according to the present invention can be carried out by using an aqueous alcohol and cyclodextrin(s).

As used herein, the "regulation of the cholesteryl ester metabolism" means the regulation of activities of formation and degradation of cholesteryl ester(s) (for example, stimulation and inhibition of the forming activity and stimulation and inhibition of the degradation activity) and defoaming, and the evaluation method" includes a method of screening an active substance, in addition to a simple method of evaluating the activity of a test substance.

In the evaluation method of the present invention, it is preferable that cells are cultured in the presence of a test substance and in the absence of the test substance (control), free cholesterol is selectively extracted by the method described above, and after this extraction, the amounts of the intracellular cholesteryl esters in respective cells cultured in the presence of and in the absence of the test substance are measured and compared to each other, but the method is not limited thereto, and the regulatory activity of a test substance on the metabolism may be evaluated by measuring and comparing e.g. the amount of extracted free cholesterol in a method known per se.

In the evaluation method of the present invention, the intracellular cholesterol and cholesteryl esters may have been labeled (for example radioactive or fluorescent label), and the cells in the extraction step may have been fixed with a suitable fixing agent (for example, aldehydes such as formaldehyde, paraformaldehyde, etc.; alcohols such as ethanol, methanol, etc.; organic solvents such as acetone, etc.; and the like).

A main object of the evaluation method of the present invention lies in evaluation and screening of substances useful as defoaming stimulators, antiatherogenic agents or regression agents of atherosclerotic lesion, wherein the cells used may be loaded with cholesterol or cholesteryl ester, or a cholesteryl ester may be formed in the cells to allow the cells to become foam cells, thereby permitting the activity of a test substance to exhibit more significantly, in order to screen compounds having an inhibitory activity on formation of intracellular cholesteryl esters, compounds having a stimulating activity on formation of intracellular cholesteryl esters, compounds having a defoaming activity, and the like.

Loading of the cells with cholesterol or a cholesteryl ester can be carried out by adding them as an alcohol solution or as a complex with lipoprotein (for example, LDL, acetyl LDL, oxidized LDL, glycated LDL, β-VLDL, etc.), and formation of a cholesteryl ester in the cells can be carried out by adding oxidized sterol(s) (for example, 25-hydroxycholesterol, 7-ketocholesterol, etc.) or lipoprotein (for example, LDL, acetyl LDL, oxidized LDL, glycated LDL, β-VLDL, etc.), cholesterol-containing liposome, cytokine such as interferon γ, and the like.

The cell strain which can be used in such loading and formation includes, for example, cells capable of incorporating cholesterol or lipoprotein and accumulating cholesterol esters in the cells, and examples of such cells include peripheral blood-derived monocyte macrophages, peritoneal macrophages, alveolar macrophages, aortic macrophages, smooth muscular cells, fibroblasts, hepatocytes and dendritic cell strains derived from mammals (humans, monkeys, pigs, rabbits, rats, mice, hamsters etc.), and cells strains such as THP-1 (human), U937 (human), HepG2 (human), J774 (mouse), L-1 (mouse), RAW264.7 (mouse), WEHI (mouse), P388D1 (mouse), etc.

Culture of these cells can be carried out in a suitable medium and under suitable culture conditions for the cells used.

Although not intended to specifically limit thereto, it can be usually judged that when the difference between the amount of intracellular cholesteryl esters in cells cultured in the presence of a test substance and the amount of intracellular cholesteryl esters in control cells cultured in the absence of the test substance is 10 % or more, preferably 20 % or more, the test substance has a regulatory activity on the cholesteryl ester metabolism.

According to the evaluation method of the present invention, the influence of a test substance on the cholesteryl ester cycle or on formation and degradation of cholesteryl esters can be directly evaluated, and those compounds having an inhibitory activity on formation of cholesteryl esters, a stimulating activity on degradation of cholesteryl esters and a defoaming activity can be efficiently selected.

The compounds having an activity of regulating the intracellular cholesteryl ester metabolism, the compounds having a defoaming activity or salts thereof (preferably pharmaceutically acceptable salts), which are selected in the manner described above, are useful as defoaming stimulators, antiatherogenic agents or regression agents of artherosclerotic lesion in mammals (humans, monkeys, pigs, rabbits, rats, mice, hamsters etc.).

Because these compounds or salts thereof have a degrading activity on lipid-rich plaques, they are useful as an agent for preventing and treating the acute coronary syndrome such as acute myocardial infarction and labile angina pectoris, peripheral arterial occlusion, and re-stricture after percutaneous transluminal coronary angioplasty (PTCA).

The pharmaceutical compositions of defoaming stimulatros, antiatherogenic agents or regression agents of artherosclerotic lesion, etc., according to the present invention can be prepared for example in the form of solid or liquid pharmaceutical compositions for oral or parenteral administration by pharmaceutical manufacturing techniques known in the art, that is, by using known excipients and carriers, and then administered into mammals. The dose can be suitably selected depending on the selected particular compounds.

More specifically, the selected compounds or salts thereof can be used orally or parenterally by injection, drip infusion, inhalation, rectal administration or topical administration, and used as it is or as a pharmaceutical composition in the form of a pharmaceutical preparation (for example, powders, granules, tablets, pills, capsules, injections, syrups, emulsions, elixirs, suspensions, solutions etc.). That is, the compound or a salt thereof can be used alone or mixed with pharmaceutically acceptable carriers (adjuvant, an excipient, a filler and/or a diluent, etc.).

These pharmaceutical compositions can be formed into pharmaceutical preparations by a conventional method. Usually, such pharmaceutical preparations can be produced by mixing/kneading the active ingredient compound with additives such as an excipient, a diluent, a carrier etc. The term parenteral used herein includes subcutaneous injection, intravenous injection, intramuscular injection, intraperitoneal injection or drip infusion.

An injectable preparation, for example, an aseptic injectable aqueous suspension or an oily suspension is prepared by a method known in the art by using a suitable dispersant, a wetting agent and a suspension agent. The aseptic injectable preparation may be, for example, an aqueous solution such as an aseptic solution or suspension which can be injected with a non-toxic diluent or solvent which can be parenterally administered. The vehicle or solvent which can be used includes water, Ringer's solution and isotonic saline. Further, an aseptic non-volatile oil can be used usually as the solvent or suspending solvent. As the non-volatile oil, any non-volatile oils and fatty acids can be used which also include naturally occurring or synthetic or semi-synthetic fatty oils or fatty acids, and naturally occurring or synthetic or semi-synthetic mono- or di-triglycerides.

Suppositories for rectal administration can be produced by mixing the medicament with non-stimulative carriers such as cocoa butter and polyethylene glycols, which are solid at ordinary temperatures but liquid at the temperature of the intestinal tract, to melt in the rectum and release the medicament.

It is also effective that the pharmaceutical preparations are combined with suitable base ingredients (e.g., butyric acid polymers, glycolic acid polymers, butyric acid-glycolic acid copolymers, a mixture of butyric acid polymers and glycolic acid polymers, polyglycerol fatty acid esters etc.), to form sustained-release preparations.

The solid pharmaceutical preparations for oral administration include those described above, such as powders, granules, tablets, pills and capsules. The pharmaceutical preparations in such forms can be produced by mixing and/or kneading the active ingredient compound with at least one additive such as sucrose, lactose, cellulose sugar, mannitol (D-mannitol), maltitol, dextran, starch (e.g., corn starch), fine crystalline cellulose, agar, alginates, xanthine, chitosan, pectin, tragacanth gum, arabic gum, gelatin, collagen, casein, albumin, synthetic or semi-synthetic polymers or glycerides. As usual, the preparations in such forms can contain additional additives such as inert diluents, lubricants such as magnesium stearate, preservatives such as parabene and sorbic acid, antioxidants such as ascorbic acid, α-tocopherol and cysteine, disintegrating agents (e.g. sodium croscarmellose), binders (for example, hydroxypropyl cellulose), thickeners, buffer agents, sweeteners, flavorings, perfumes etc. The tablets and pills can also be produced with an enteric coating. The liquids for oral administration include pharmaceutically acceptable emulsions, syrups, elixirs, suspensions and solutions, which may contain inert diluents used ordinarily in this field, for example water and additives if necessary. These liquids for oral administration can be produced in a conventional method of mixing the active ingredient compound with inert diluents and other additives if necessary. Depending on the preparation form, the oral preparation is compounded with the active ingredient compound in an amount of usually about 0.01 to 99% by weight, preferably about 0.1 to 90% by weight, normally about 0.5 to 50% by weight.

The amount of the active ingredient compound administered into a certain specific patient is determined depending on the age, weight, general health conditions, sex, diet, administration time, administration method, excretion rate, combination of medicaments, the morbid state of the patient during treatment, or inconsideration of these and other factors.

The amount of the pharmaceutical composition administered per day is varied depending on the condition and body weight of a patient, the type of the compound, administration route etc., and for example, when the pharmaceutical composition is used as an atherosclerosis inhibitor, the amount of the active ingredient administered per day into a person (weighing about 60 kg) is about 1 to 500 mg, preferably about 10 to 200 mg in the case of the oral agent or about 0.1 to 100 mg, preferably about 1 to 50 mg, usually 1 to 20 mg in the case of the parenteral agent.

In treatment of these diseases, the selected compound or a salt thereof may be used alone in treatment or in combination with other pharmaceutical ingredients including an agent for reducing lipid or an agent for reducing cholesterol, an agent for protecting the myocardium, an agent for treating a coronary disease, an agent for treating diabetes, an agent for treating hypothyroidism, an agent for treating the nephrotic syndrome and an agent for treating chronic renal insufficiency, and in this case, these compounds are administered preferably as oral preparations or if necessary in the form of suppositories as rectal preparations. In this case, possible combined ingredients include for example fibrates [e.g., clofibrate, benzafibrate, gemfibrozil, etc.]; nicotinic acid, its derivatives and analogues [e.g., acipimox and probucol]; bile acid binding resins [e.g., cholestyramine, cholestipol, etc.]; compounds suppressing cholesterol uptake [e.g., sitosterol, neomycin, etc.]; compounds inhibiting biosynthesis of cholesterol [e.g., HMG-CoA reductase inhibitors such as lovastatin, simvastatin, pravastatin, etc.]; and squalene synthase inhibitors [e.g., benzoxazepine derivatives described in EP 0567026, WO 97/10224, etc.]; and squalene epoxidase inhibitors [e.g., NB-598 and its analogous compounds, etc.].

Other possible combined ingredients include oxidosqualene-lanosterol cyclase, for example decalin derivatives, azadecalin derivatives and indan derivatives.

Further, the following ingredients may be used in combination and preferably by oral administration:
Diabetic drugs: actos, rosiglitazone, kinedak, penfill, humalin, euglucon, glimicron, daonil, novolin, monotard, insuline family, glucobay, dimelin, rastinone, bacilcon, deamelin S, Iszilin family;
Anti-hypothyroidism drugs: dry thyroid (Thyreoid), levothyroxine sodium (Tyradin S), liothyronijine sodium (thyronine, tyronine);
Nephrotic syndrome drugs: prednisolone (Predonine), prednisolone sodium succinate (Predonine), methylprednisolone sodium succinate (Solu medrol), betamethasone (Rinderon);
Anticoagulation therapeutic drugs: dipyridamole (Persantine), dilazep hydrochloride (Comelian), ticlopidine, clopidogrel, Xa inhibitor;
Chronic renal failure drugs: diuretic drugs [e.g., furosemide (Lasix), bumetanide (Lunetoron), azosemide (Diart)], antihypertensive drugs [e.g., ACE inhibitors (enalapril maleate (Renivace)] and Ca antagonists (manidipine), α-receptor blockers, All antagonists (candesartan), etc.

Further, the selected compound or a salt thereof has a regressing activity on lipid-rich plaques, and thus it is also useful as an agent for preventing and treating thrombosis. It can be used alone or in combination with the following known therapeutic drugs and used preferably by oral administration.

Thrombogenesis preventive and therapeutic drugs:
anticoagulant drugs [e.g., heparin sodium, heparin calcium, warfarin calcium (Warfarin), Xa inhibitor], thrombolytic drugs [e.g., tPA, urokinase], antiplatelet drugs [e.g., aspirin, sulfinpyrazone (Anturan), dipyridamole (Persantine), ticlopidine (Panaldine), cilostazol (Pletal), GPIIb/IIIa antagonist (ReoPro)];
Coronary vasodilators: nifedipine, diltiazem, nicorandil, nitrite drugs;
Myocardial protective drugs: heart ATP-K opener, endothelin antagonist, urotensin antagonist.

Hereinafter, the present invention is illustrated in more detail by reference to Examples and Formulation Examples, but the present invention is not limited thereto.

### Example 1

### Selective removal of intracellular free cholesterol with aqueous alcohol

According to the method of Hakamada et al. ("Jikken Igaku (Experimental Medicine), Extra Issue", vol. 14, No. 12, Circulation Study Protocol, pp. 49-52 (1996)), peritoneal macrophages were prepared from C57BL6J mice stimulated with thioglycolate, and then cultured for 24 hours in RPMI 1640-25 mM HEPES (pH 7) containing β-very low-density lipoprotein (β-VLDL, 150 µg cholesterol/ml) prepared according to the method of Ishii et al. (Arterioscler. Thromb., 12, 1139-1145, 1992), a conjugate of 0.2 mM oleic acid/0.2% bovine serum albumin, and [1,2,6,7-³H]-cholesterol (NEN, 37 KBq/ml) to obtain foam cells in which cholesteryl esters were accumulated. The foam cells were extracted with 80% aqueous methanol for 10 minutes and then the whole lipid was extracted with hexane : isopropanol (3 : 2). A part of the extract was fractionated by the TLC method (Merck Silica Gel Plate No. 5582, developing solvent: petroleum ether/diethyl ether/acetic acid = 9 : 1 : 0.1), free cholesterol and cholesteryl ester fractions were cut off, a liquid scintillator was added, and the radioactivity thereof was measured.

The results are shown in Table 1.

### Example 2

### Selective removal of intracellular free cholesterol by cyclodextrin

The foam mouse macrophages prepared in Example 1 were fixed at room temperature for 10 minutes with 10 % formalin and then extracted with 2% aqueous methyl β-cyclodextrin (Sigma) for 4 hours (2 hours, twice), and the whole lipid were extracted with hexane : isopropanol (3 : 2). A part of the extract was fractionated by the TLC method (Merck Silica Gel Plate No. 5582, developing solvent: petroleum ether/diethyl ether/acetic acid = 9 : 1 : 0.1), free cholesterol and cholesteryl ester fractions were cut off, a liquid scintillator was added, and the radioactivity thereof was measured.

The results are shown in Table 1.

**Table 1**

| Removal of free cholesterol by aqueous alcohol and cyclodextrin | | |
|---|---|---|
| | Radioactivity/well | |
| Extraction method | free cholesterol | cholesteryl esters |
| Control | 46418 ± 9560 | 52018 ± 379 |
| 80% methanol | 2162 ± 312 | 49141 ± 387 |
| Control | 821549 ± 478 | 85377 ± 20866 |
| 2% methyl β-cyclodextrin | 3059 ± 560 | 99414 ± 3777 |

In Table 1, the control is the measurements of free cholesterol and cholesteryl esters in the cells not subjected to extraction with aqueous methanol or methyl β-cyclodextrin.

As shown in Table 1, the amount of free cholesterol in the cells subjected to extraction with aqueous methanol or methyl β-cyclodextrin was reduced to 4 to 5 % of the control, and the ratio thereof to the total cholesterol was also as very low as 3 to 5 %. Then, since almost all cholesterol fractions remaining in the cells subjected to extraction with aqueous methanol or methyl (β-cyclodextrin are cholesteryl esters, it is possible to readily evaluate the amount of cholesteryl esters in the cells by measuring the residual radioactivity in the cells. That is, this method enables one to efficiently search for compounds influencing the intracellular cholesteryl ester cycle or formation of cholesteryl esters or degradation of cholesteryl esters.

### Example 3

### Measurement of regulatory activity on intracellular cholesteryl ester cycle

This example illustrates an example of an activity measurement system for a substance regulating the intracellular cholesteryl ester cycle.

Dibutyryl cyclic AMP (hereinafter, sometimes referred to as dbc-AMP; Sakr SW et al., Biochemica et Biophysica Acta 1438, 85-98, 1999; Bernard DW et al., J. Biol. Chem., 266, 710-716, 1991), which is well-known as a compound exerting an influence on the cholesteryl ester cycle in cells, was used as a test compound to carry out this measurement system.

In an RPMI 1640-HEPES medium (pH 7) containing the test compound, the foam macrophages prepared in Example 1 were cultured for 24 hours. The cells were extracted with 80% aqueous methanol for 10 minutes and then solubilized with 0.1 NaOH/0.1% SDS solution, a liquid scintillator was added to a portion thereof, and its radioactivity was measured. Each value of radioactivity was corrected by the amount of cell protein measured with a BCA assay kit (Pierce Ltd.), and assuming that the radioactivity of a control group (in the absence of the test compound) was 100%, the intracellular cholesteryl ester content (%) of a group treated with the compound was calculated.

The results are shown in Table 2.

**Table 2**

| Regulation of cholesteryl ester metabolism by dibutyryl cyclic AMP | |
|---|---|
| | Cholesteryl ester content (%) |
| Control | 100 ± 15 |
| 100µM dbc-AMP | 60 ± 12 p < 0.01* |

| | |
|---|---|
| *Student's t-test (N=3) | |

As shown in Table 2, it was revealed that dibutyryl cyclic AMP exhibits a cholesteryl ester cycle-regulating action attributable to its potent cholesteryl ester-hydrolytic activity.

### Example 4

### Confirmation of promoting activity of dibutyryl cyclic AMP on defoaming

The activity of the test compound on defoaming was measured by treating the foam mouse macrophages prepared in Example 1, with RPMI 1640-25 mM HEPES medium (pH 7) containing the test compound for 24 hours, followed by adding 20% fetal bovine serum (JRH Ltd.), and determining the radioactivity released into the medium after further culture for 24 hours. The defoaming-promoting activity of a group treated with the compound was calculated as a ratio (%) of the radioactivity corrected by a blank value to that of a control group. The blank value used was the radioactivity in the absence of the test compound and 20% fetal bovine serum. The defoaming-promoting activities of the control group and the group treated with the compound were corrected by the amount of cell protein measured by the BCA assay kit from Pierce Ltd. after the cells were solubilized with 0.1 N NaOH/0.1 % SDS solution.

The results are shown in Table 3.

### Example 5

### Confirmation of influence of dibutyryl cyclic AMP on intracellular cholesteryl ester content

The mouse peritoneal macrophages prepared in Example 1 were cultured for 24 hours in an RPMI 1640-25 mM HEPES medium (pH 7) containing β-VLDL (150 µg/ml) and a conjugate of 0.2 mM oleic acid/0/2% bovine serum albumin to obtain foam cells in which cholesteryl esters were accumulated. These foamed macrophages were treated with an RPMI 1640-25 mM HEPES medium (pH 7) containing the test compound for 24 hours, and then the whole lipid was extracted. The lipid thus extracted was dissolved in isopropanol and measured for the amounts of the whole cholesterol and free cholesterol by the enzyme method (E-Test Wako, Wako Ltd.), and, from the difference therebetween, the amount of cholesteryl esters was calculated. The amounts of cell protein in a control group and a group treated with the compound were measured with a BCA assay kit from Pierce Ltd. after the cells were solubilized with 0.1 N NaOH/0.1% SDS solution, and assuming that the amount of cholesteryl esters (µg/mg cell protein) in the cells in the control group was 100%, the amount of cholesteryl esters (%) in the group treated with the compound was calculated.

The results are shown in Table 4.

**Table 3**

| Action of dibutyryl cyclic AMP in promoting cholesterol efflux | |
|---|---|
| | Amount of cholesterol efflux (%) |
| Control | 100 ± 9 |
| 100µM dbc-AMP | 160 ± 15 p < 0.01* |

| | |
|---|---|
| *Student's t-test (N=3) | |

**Table 4**

| Action of dibutyryl cyclic AMP in reducing cholesteryl ester content | |
|---|---|
| | Cholesteryl ester content(%) |
| Control | 100 ± 9 |
| 100µM dbc-AMP | 78 ± 4 p < 0.01* |

| | |
|---|---|
| *Student's t-test (N=3) | |

Using a conventional measurement method, dibutyryl cyclic AMP was examined for its action of transferring cholesterol from the foam macrophages in Example 4 and for its influence on the intracellular cholesteryl ester content in Example 5.

The result reveals that dibutyryl cyclic AMP exerts an action both on transfer of cholesterol (Table 3) and on reduction of the cholesteryl ester content (Table 4). Because the foam cells are main components constituting atherosclerotic lesions, compounds found in the searching system of the present invention are useful as novel agents for treating atherosclerosis by inhibiting formation and stimulating regression of atherosclerotic lesions.

### Example 6

### Inhibitory action of 2-[6-chloro-4-(2-methylphenyl)-2-oxo-2H-chromene-3-yl]-N-(2,6-dimethoxyphenyl) acetamide on synthesis of intracellular cholesteryl esters

Mouse peritoneal macrophages prepared in the method described in Example 1 were cultured for 24 hours in an RPMI 1640-25 mM HEPES medium (pH 7) containing 1% fetal bovine serum and [1,2,6,7-³H]-cholesterol (37 Kbq/ml, NEN Ltd.). The macrophages were labeled with ³H-cholesterol and then cultured for 24 hours in an RPMI 1640-25 mM HEPES medium (pH 7) containing 2-[6-chloro-4-(2-methylphenyl)-2-oxo-2H-chromene-3-yl]-N-(2,6-dimethoxyphenyl) acetamide (referred to hereinafter as Compound A), 25-hydroxycholesterol (5 µg/ml, Sigma) and 0.2 mM oleic acid/0.2 % bovine serum albumin, and the content of synthesized intracellular cholesteryl esters was quantified by the TLC fractionation method or the aqueous methanol extraction method (hereinafter also referred to as the methanol method). The measurement by the TLC fractionation method was conducted by extracting the whole intracellular lipid with a hexane-isopropanol mixture (3 : 2, v/v) and then fractionating the lipid in TLC under the same conditions as in Example 1 to quantify the intracellular cholesteryl esters. The measurement by the aqueous methanol extraction method was conducted by treating the cells with 80% methanol for 10 minutes, then solubilizing the cells with 0.1 N NaOH/0.1% SDS solution, and measuring their radioactivity. Each value of radioactivity was corrected by the amount of cell proteins measured with a BCA assay kit (Pierce Ltd.) after the cells were solubilized with 0.1 N NaOH/0.1% SDS solution, and assuming that the radioactivity (dpm/mg cell protein) of the control group in the absence of the test compound was 100%, the content of intracellular cholesteryl esters (%) in the group treated with Compound A was calculated.

The results are shown in Table 5.

**Table 5**

| Inhibitory action on synthesis of cholesteryl esters | | | | |
|---|---|---|---|---|
| | Content of intracellular cholesteryl esters(control group = 100 %) | | | |
| | Methanol method | | TLC method | |
| Concentration of Compound A | Mean | SD | Mean | SD |
| Control group | 100 | 2 | 100 | 5 |
| 0.01mM | 41 | 2 | 44 | 2 |
| 0.03mM | 19 | 1 | 16 | 2 |
| 0.1mM | 9 | 0.2 | 5 | 1 |
| n =3 | | | | |

### Example 7

### Promoting action of Compound A on decomposition of intracellular cholesteryl esters

Foam cells derived from mouse peritoneal macrophages, prepared in the method described in Example 1, were cultured for 48 hours in an RPMI 1640-25 mM HEPES medium (pH 7) containing Compound A, and a reduction in the intracellular cholesteryl ester content was quantified by a TLC fractionation method or an aqueous methanol extraction method. The measurement by the TLC fractionation method was conducted by extracting the whole intracellular lipid with a hexane-isopropanol mixture (3 : 2, v/v) and then fractionating the lipid by TLC under the same conditions as in Example 1 to quantify the intracellular cholesteryl esters. The measurement by the aqueous methanol extraction method was conducted by treating the cells with 80% methanol for 10 minutes, then solubilizing the cells with 0.1 N NaOH/0.1 % SDS solution, and measuring their radioactivity. Each value of radioactivity was corrected by the amount of cell proteins measured with a BCA assay kit (Pierce Ltd.) after the cells were solubilized with 0.1 N NaOH/0.1% SDS solution, and assuming that the radioactivity (dpm/mg cell protein) of the control group in the absence of the test compound was 100%, the content of intracellular cholesteryl esters (%) in the group treated with Compound A was calculated.

The results are shown in Table 6.

**Table 6**

| Stimulating activity of degradation of cholesteryl esters | | | | |
|---|---|---|---|---|
| | Content of intracellular cholesteryl esters (control group = 100 %) | | | |
| | Methanol method | | TLC method | |
| Concentration of Compound A | Mean | SD | Mean | SD |
| Control group | 100 | 2 | 100 | 10 |
| 0.01mM | 74 | 3 | 71 | 3 |
| 0.1mM | 14 | 1 | 8 | 1 |
| n =3 | | | | |

By examining the influence of Compound A on an activity of synthesizing cholesteryl esters and an activity of degradation of cholesteryl esters in the cells (Tables 5 and 6), it was found that Compound A has a potent inhibitory activity on synthesis of cholesteryl esters and a strong promoting activity on degradation of cholesteryl esters. Further, the results of the TLC fractionation method, i.e., a conventional method were well correlated with those of the method of the present invention (aqueous alcohol extraction method), thus proving the usefulness of the method of the present invention.

### Example 8

The promoting action of Compound A on defoaming was measured by using the method described in Example 4.

The results are shown in Table 7.

**Table 7**

| Promoting action on defoaming | | | |
|---|---|---|---|
| Concentration of Compound A | Amount of transferred (control group = 100 %) | cholesterol | |
| | Mean | SD | t-test |
| Control group | 100 | 18 | |
| 0.01mM | 142 | 2 | p<0.05 |
| 0.1mM | 191 | 10 | p<0.01 |

### Example 9

### Action of Compound A on reduction of the content of intracellular cholesteryl esters

The action of Compound A on reduction of the content of intracellular cholesteryl esters was measured by using the method described in Example 5.

The results are shown in Table 8.

**Table 8**

| Action on reduction of intracellular cholesteryl ester content | | | |
|---|---|---|---|
| | Content of intracellular cholesteryl esters (control group = 100 %) | | |
| Concentration of Compound A | Mean | SD | t-test |
| Control group | 102 | 2 | |
| 0.01mM | 63 | 3 | p<0.05 |
| 0.1mM | 48 | 4 | p<0.01 |
| n =3 | | | |

By examining the action of Compound A on transfer of cholesterol from the foamed macrophages and on the content of intracellular cholesteryl esters, it was found that Compound A has a strong action both on cholesterol efflux (Table 7) and on reduction of the content of cholesteryl esters (Table 8). Because the foamed cells are main components constituting arteriosclerotic lesions, Compound A is useful as a novel agent for treating atherosclerosis by inhibiting formation and stimulating regression of atherosclerotic lesions.

| Formulation Example 1 Capsules | |
|---|---|
| (1) Compound A | 10 mg |
| (2) Lactose | 90 mg |
| (3) Microcrystalline cellulose | 70 mg |
| (4) Magnesium stearate | 10 mg |
| 1 capsule | 180 mg |

The whole amounts of (1), (2) and (3) and 1/2 of (4) were kneaded, and then granulated. The remainder of (4) was added thereto and the whole kneaded product was sealed into gelatin capsules to give capsules.

| Formulation Example 2 Tablets | |
|---|---|
| (1) Compound A | 10 mg |
| (2) Lactose | 35 mg |
| (3) Corn starch | 150 mg |
| (4) Microcrystalline cellulose | 30 mg |
| (5) Magnesium stearate | 5 mg |
| 1 tablet | 230 mg |

The whole amounts of (1), (2) and (3), 2/3 of (4) and 1/2 of (5) were kneaded, and then granulated. The remainders of (4) and (5) were added to the granules and then pressure-molded into tablets.

| Formulation Example 3 Injections | |
|---|---|
| (1) Compound A | 10 mg |
| (2) Inositol | 100 mg |
| (3) Benzyl alcohol | 20 mg |
| 1 ampoule | 130 mg |

The whole amounts of (1), (2) and (3) were dissolved in distilled water for injection such that the whole volume became 2 ml, and the solution was sealed into an ampoule. All the procedures were conducted under germ-free conditions.

### Industrial Applicability

According to the method of extracting or removing intracellular free cholesterol, the method of evaluating an activity of regulating the intracellular cholesteryl ester metabolism, etc. of the present invention, a defoaming promoter, an atherosclerosis inhibitor or an atherosclerotic lesion regression agent can be selected.

## Claims

1. A method of extracting or removing intracellular free cholesterol which comprises selectively extracting or removing free cholesterol from cells with an aqueous alcohol.

2. The extracting or removing method according to claim 1, wherein the alcohol is a polar aliphatic monohydric alcohol having 1 to 4 carbon atoms.

3. The extracting or removing method according to claim 1, wherein the alcohol is methanol.

4. The extracting or removing method according to claim 1, wherein the water content of the aqueous alcohol is 5 to 60 (v/v) %.

5. The extracting or removing method according to claim 1, wherein the aqueous alcohol and cyclodextrin(s) are used.

6. The extracting or removing method according to claim 1, wherein the cells are those derived from a mammal.

7. The extracting or removing method according to claim 1, wherein the cells are peripheral blood-derived monocyte macrophages derived from a mammal, peritoneal macrophages derived from a mammal, alveolar macrophages derived from a mammal, aortic macrophages derived from a mammal, smooth muscular cells derived from a mammal, fibroblasts derived from a mammal, hepatocytes derived from a mammal, dendritic cell strains derived from a mammal, THP-1 (human), U937 (human), HepG2 (human), J774 (mouse), L-1 (mouse), RAW264.7 (mouse), WEHI (mouse) or P388D1 (mouse).

8. A method of measuring the amount of intracellular cholesteryl esters which comprises selectively extracting and removing free cholesterol from cells with an aqueous alcohol and/or cyclodextrin(s) and then measuring the amount of the intracellular cholesteryl esters.

9. The method according to claim 8, wherein whole lipid is extracted with a solvent from the cells from which free cholesterol has been extracted and removed, and the amount of cholesteryl esters in the whole lipid is quantified by an enzyme method using a cholesterol oxidase, a TLC method or liquid chromatography.

10. The method according to claim 9, wherein the solvent is chloroform-methanol or hexane-isopropanol.

11. The method according to claim 8, wherein the intracellular cholesterol is labeled, free cholesterol is selectively extracted and removed from the cells, and then the amount of the label in the cells is measured.

12. A method of evaluating the regulatory activity of a test substance on the intracellular cholesteryl ester metabolism which comprises the procedure of selectively extracting free cholesterol from cells with an aqueous alcohol.

13. The evaluation method according to claim 12, wherein (i) the cells are loaded with cholesterol or a cholesteryl ester, or (ii) a cholesteryl ester are formed in the cells to allow the cells to become foam cells.

14. The evaluation method according to claim 13, wherein the loading of cells with cholesterol or a cholesteryl ester is carried out by addition of (i) a solution of cholesterol or a cholesteryl ester in an alcohol or (ii) a complex of cholesterol or a cholesteryl ester and lipoprotein.

15. The evaluation method according to claim 14, wherein the lipoprotein is LDL, acetyl LDL, oxidized LDL, glycated LDL or β-VLDL.

16. The evaluation method according to claim 13, wherein the formation of the cholesteryl ester in the cells is carried out by addition of oxysterols, lipoprotein, cholesterol-containing liposome or cytokine.

17. The evaluation method according to claim 16, wherein the oxysterol is 25-hydroxycholesterol or 7-ketocholesterol.

18. The evaluation method according to claim 16, wherein the lipoprotein is LDL, acetyl LDL, oxidized LDL, glycated LDL or β-VLDL.

19. The evaluation method according to claim 16, wherein the cytokine is interferon γ.

20. The evaluation method according to claim 12, wherein the alcohol is a polar aliphatic monohydric alcohol having 1 to 4 carbon atoms.

21. The evaluation method according to claim 12, wherein the alcohol is methanol.

22. The evaluation method according to claim 12, wherein the water content of the aqueous alcohol is 5 to 60 (v/v) %.

23. The evaluation method according to claim 12, wherein the cells are those derived from a mammal.

24. The evaluation method according to claim 12, wherein the cells are peripheral blood-derived monocyte macrophages derived from a mammal, peritoneal macrophages derived from a mammal, alveolar macrophages derived from a mammal, aortic macrophages derived from a mammal, smooth muscular cells derived from a mammal, fibroblasts derived from a mammal, hepatocytes derived from a mammal, dendritic cell strains derived from a mammal, THP-1 (human), U937 (human), HepG2 (human), J774 (mouse), L-1 (mouse), RAW264.7 (mouse), WEHI (mouse) or P388D1 (mouse).

25. The evaluation method according to claim 12, wherein free cholesterol is selectively extracted with an aqueous alcohol from cells cultured in the presence of a test substance and cells cultured in the absence of the test substance, and then the amounts of intracellular cholesteryl esters in respective cells cultured in the presence of and in the absence of the test substance are measured and compared to each other.

26. The evaluation method according to any one of claims 12 to 25, wherein the regulation of the intracellular cholesteryl ester metabolism is regulation of an activity of forming intracellular cholesteryl esters.

27. The evaluation method according to any one of claims 12 to 25, wherein the regulation of the intracellular cholesteryl ester metabolism is regulation of an activity of hydrolysis of intracellular cholesteryl esters.

28. The evaluation method according to any of claims 12 to 25 which is a method of screening a compound having an activity of regulating the intracellular cholesteryl ester metabolism.

29. The evaluation method according to claim 28 which is a method of screening a compound having an inhibitory activity on formation of intracellular cholesteryl esters.

30. The evaluation method according to claim 28 which is a method of screening a compound having a promoting activity on hydrolysis of intracellular cholesteryl esters.

31. The evaluation method according to claim 28 which is a method of screening a compound having a defoaming activity.

32. A compound having a regulatory activity on the intracellular cholesteryl ester metabolism selected by the method according to claim 28, or a salt thereof.

33. A defoaming stimulator which comprises as an active ingredient a compound having a regulatory activity on the intracellular cholesteryl ester metabolism selected by the method according to claim 28, or a pharmaceutically acceptable salt thereof.

34. Antiatherogenic agents or regression agents of atherosclerotic lesion which comprises as an active ingredient a compound having a regulatory activity on the intracellular cholesteryl ester metabolism selected by the method according to claim 28, or a pharmaceutically acceptable salt thereof.

35. A method of stimulating defoaming which comprises administering an effective amount of a compound having a regulatory activity on the intracellular cholesteryl ester metabolism selected by the method according to claim 28, or a salt thereof to a mammal.

36. A method of preventing and treating atherosclerosis which comprises administering an effective amount of a compound having a regulatory activity on the intracellular cholesteryl ester metabolism selected by the method according to claim 28, or a salt thereof to a mammal.

37. Use of a compound having a regulatory activity on the intracellular cholesteryl ester metabolism selected by the method according to claim 28, or a salt thereof for the manufacture of a defoaming stimulator.

38. Use of a compound having a regulatory activity on the intracellular cholesteryl ester metabolism selected by the method according to claim 28, or a salt thereof for the manufacture of an agent for preventing and treating atherosclerosis.

39. Use of an aqueous alcohol for selectively extracting or removing free cholesterol from cells.
